# EUROPEAN PATENT SPECIFICATION

(11) **EP 1 630 934 B1**
(45) Date of publication and mention of the grant of the patent: **28.04.2010**
(21) Application number: 04029337.5
(22) Date of filing: 10.12.2004
(51) Int. Cl.: H02K 13/00, H02K 5/14, H01R 39/41

(54) **Brush holder assembly of alternator for vehicle**
Bürstenhalteranordnung für einen Fahrzeuggenerator
Ensemble porte-balais pour véhicule automobile

(30) Priority: 31.08.2004 KR 2004069043
(43) Date of publication of application: 01.03.2006
(73) Proprietor: VALEO ELECTRICAL SYSTEMS KOREA LTD., Kyongju-si, Kyongsangbuk-do (KR)
(72) Inventor: Lee, Hyun-Cheol, Dong-gu Daegu-si, 701-032 (KR)
(74) Representative: TER MEER - STEINMEISTER & PARTNER GbR

(56) References cited:
- US-A- 2 798 176
- US-A- 4 705 983
- US-A- 5 550 418
- US-A1- 2002 105 242

## Description

### BACKGROUND OF THE INVENTION

### 1. Field of the Invention

The present invention relates to a brush holder assembly of an alternator for a vehicle according to the preamble of claim 1. In particular the invention relates to, a brush holder assembly of an alternator for a vehicle, in which the structure of a brush connecting rod extended from an end of a brush is modified to be connected with a terminal piece and sealed, thereby improving the assembling workability and largely enhancing the sealing performance.

### 2. Description of the Background Art

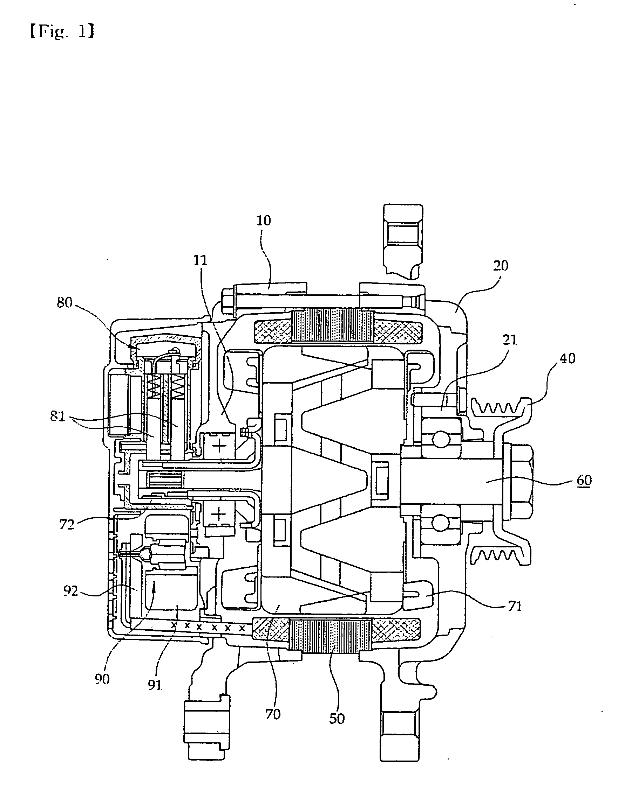

Generally, as shown in Figure 1, an alternator for a vehicle includes a casing 30 formed of a rear bracket 10 and a front bracket 20 respectively having shaft support parts 11 and 21, a shaft 60 installed in the interior of the casing and fixed with a pulley 40 at one end, a rotor 70 fixed at the shaft, a fan 71 fixed at both sides of the rotor, a stator 50 fixed at an inner wall of the casing, a slip ring 72 fixed at the other end of the shaft to be electrically connected with the rotor 70, a pair of brushes 81 operatively contacting with the slip ring, a brush holder assembly 80 that receives the brushes therein, a rectifier assembly 90 electrically connected with the stator, a heat sink 91 mounted on the brush holder assembly, and a regulator 92 adapted to adjust the magnitude of an AC voltage generated in the stator in a state that it contacts the heat sink.

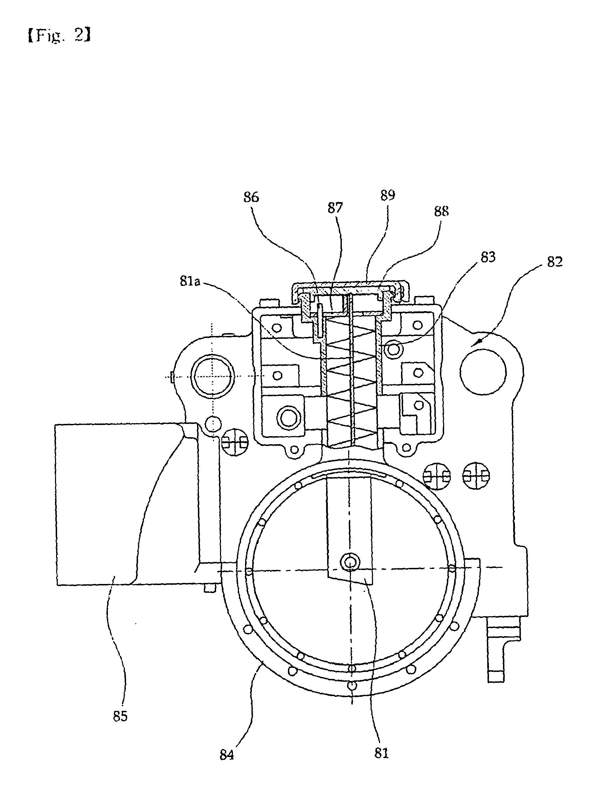

As shown in Figure 2, the brush holder assembly is formed of the brush holder 82. The brush holder 82 includes a brush box 83 into which the brushes 81 are inserted, a slip ring insertion part 84 into which the slip ring 72 is inserted, and a connecting part 85 connected with a cable.

In addition, the brushes 81 are inserted in the brush box 83, wherein lower ends of the brushes contact the surface of the slip ring 72 in the slip ring insertion part 84, and upper ends of the brushes respectively include a lead line 81 a fitted with a spring 81 b thereon. The lead lines 81 a are extended upwardly and are connected with electrode pieces 86 insert injection-molded at the holder 82.

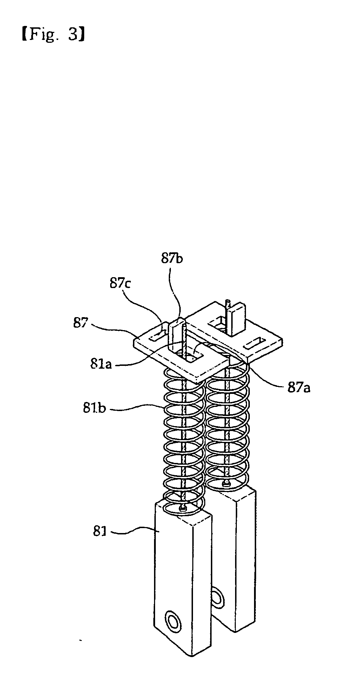

As shown in Figure 3, when the lead lines 81 a are connected with the electrode pieces 86 by means of additional metal connection pieces 87.

The metal connection pieces 87 respectively include a lead line hole 87a through which a corresponding one of the lead lines 81 a passes, a soldering connection part 87b bent from the hole, and an electrode piece hole 87a into which a corresponding one of the electrode pieces 86 is inserted. When the lead lines 81a are inserted into the lead line holes 87a, the ends of the same are connected with the soldering connection parts 87b, and the electrode pieces 86 are inserted into the electrode piece holes 87c.

When the lead lines 81 a of the both brushes are connected, a rubber lid 88 is inserted into an upper side of the brush box 83 for sealing the same, and then a synthetic resin cover 89 covers the rubber lid.

However, the conventional brush holder assembly has disadvantages that the two metal connection pieces 87 should be used for each of the brushes individually, and the electrode pieces 86 should be precisely inserted into the electrode pieces 87c, which are very small-sized. Therefore, the assembling process becomes much complicated and, accordingly, the workability becomes significantly decreased.

Further, the conventional brush holder assembly still has a disadvantage that the rubber lid 88 should be separately designed depending on the size of the brush box 83. Therefore, the sealing property decreases.

US 5,550,418 describes an alternator for an automobile according to the preamble of claim 1 having a first frame, a second frame, a stator, a shaft, a rotor, a current supply system and fans. The shaft is rotatably coupled to the first frame and the second frame, and extends outwardly beyond the first frame. The current supply system has a pair of slip rings, a pair of brushes and a brush holder. The slip rings are fixed to the shaft, and are electrically connected to a coil of the rotor. The brushes are urged against and in slidable contact with the slip rings, respectively. The brush holder is fixed to the first frame for retaining the brushes; and has a slip ring shield portion for covering and protecting the slip rings. The slip ring shield portion has openings which allow dust produced by the wearing of the brushes to be blown by the action of fans.

### SUMMARY OF THE INVENTION

Accordingly, it is an object of the present invention to provide a brush holder assembly of an alternator for a vehicle, which overcomes the problems encountered in the conventional art.

It is another object of the present invention to provide a brush holder assembly of an alternator for a vehicle capable of preventing the penetration of moisture and foreign substances by simplifying the connection of lead lines of brushes extended onto a brush box and realizing the perfect sealing of the brush box.

To achieve the above objects, there is provided a brush holder assembly of an alternator for a vehicle according to claim 1 that comprises a brush holder that includes a circular slip ring insertion part, a brush box connected on the slip ring insertion part and having a pair of brush insertion holes and electrode pieces inside, and a connection part connected at one side of the brush box; a pair of brushes that are inserted into the brush box of the brush holder, and provided with lead lines at rear ends, wherein the lead lines are provided with compression rings and are connected to the electrode pieces; a non-metallic plate is provided on the brush insertion holes which non-metallic plate having lead line holes for passing the lead lines and grommets provided to the lead line holes, wherein the non-metallic plate induces the lead lines of the brushes into the insulation state with respect to the electrode pieces provided at a side of the brush box; a silicone member adapted to sealingly cover an upper side of the non-metallic plate for blocking the brush insertion holes of the brush box; and a cover for covering an upper side of the non-metallic plate to be fixed at the brush box.

The grommets are formed in the horn shape of which a lower side is wide, and an upper side is narrow, so that the lead lines can be easily inserted and lifted.

### BRIEF DESCRIPTION OF THE DRAWINGS

The present invention will become better understood with reference to the accompanying drawings which are given only by way of illustration and thus are not limitative of the present invention, wherein;
Figure 1 is a cross sectional view illustrating a conventional brush holder of an alternator for a vehicle;
Figure 2 is a cross sectional view illustrating the construction of the conventional brush holder;
Figure 3 is a disassembled perspective view illustrating connection parts of conventional brush holder terminals;
Figure 4 is a perspective view illustrating the construction of a brush holder according to a preferred embodiment of the present invention;
Figure 5 is a front cross sectional view illustrating the construction of a brush holder according to a preferred embodiment of the present invention;
Figure 6 is an enlarged cross sectional view illustrating principal parts of the brush holder according to a preferred embodiment of the present invention; and
Figure 7 is a disassembled perspective view illustrating the principal parts of the brush holder according to a preferred embodiment of the present invention.

### DETAILED DESCRIPTION OF THE PREFERRED EMBODIMENTS

The construction and assembling process according to the present invention will be described in more detail with reference to the accompanying drawing.

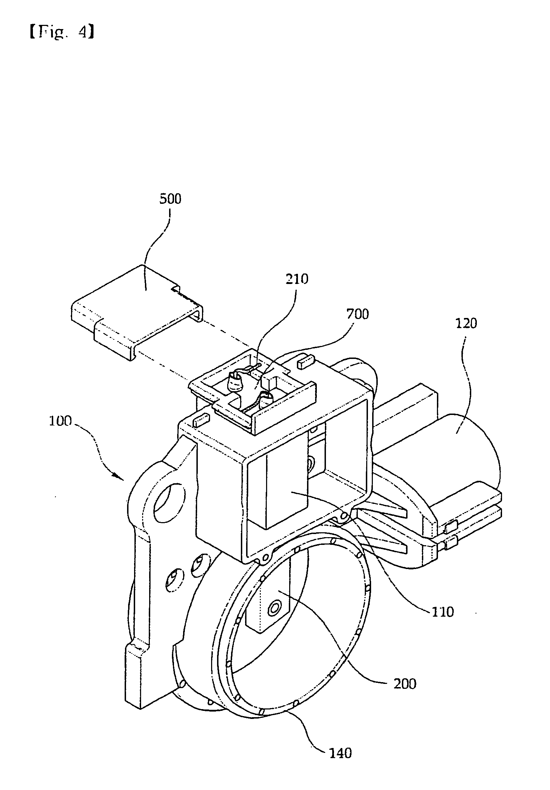

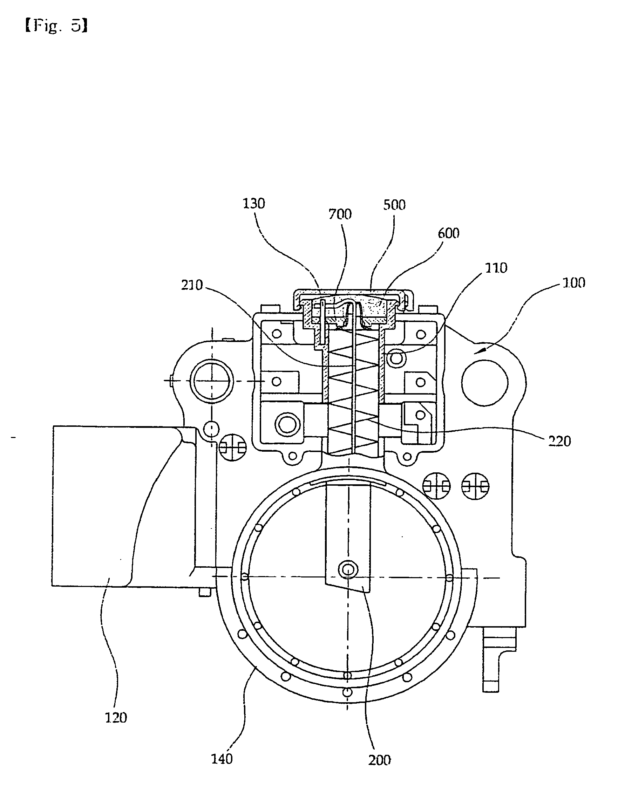

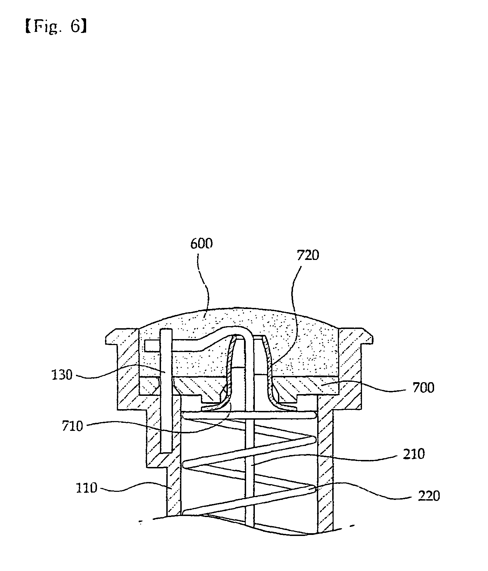

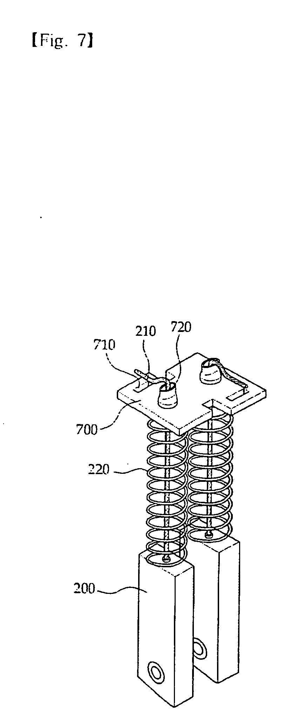

Figure 4 is a perspective view illustrating the construction of a brush holder according to the present invention, Figure 5 is a front cross sectional view illustrating the construction of a brush holder according to the present invention, Figure 6 is an enlarged cross sectional view illustrating principal parts of the brush holder according to the present invention, and Figure 7 is a disassembled perspective view illustrating principal parts of the brush holder according to the present invention.

As shown in Figures 4 and 5, a brush holder 100 includes a circular slip ring insertion part 140, a brush box 110 connected on the circular slip ring insertion part 140 and having a pair of brush insertion holes 150 and electrode pieces 130 inside, and a connection part 120 connected with one side of the brush box 110.

A pair of brushes 200 is inserted into the brush box 110 of the brush holder 100, and lead lines 210 at rear ends of the brushes are provided with compression springs in the same manner as the conventional art.

A non-metallic plate 700 is provided on the brush insertion holes 150, and the lead lines 210 of the brushes are arranged in an insulation state with respect to the electrode pieces 130 provided at one side of the brush box 110.

Namely, the non-metallic plate 700 includes lead line holes 710 for passing the lead lines 210, and grommets 720 provided at the lead line holes.

As shown in Figure 6, the grommets 720 are formed in the horn shape of which an upper side is narrow, and a lower side is wide, so that the lead lines 210 are easily inserted and lifted but not to be escaped from the lead line holes downwardly.

Therefore, ends of the lead lines 210 are welded to the electrode pieces 130 after being inserted and lifted via the grommets upwardly.

Therefore, in the present invention, silicone 600 is covered on top of the non-metallic plate 700 and is cured after the lead lines 210 are soldered.

Thereafter, a cover 500 covers the top part of the non-metallic plate 700 and is fixed at the brush box 110, thereby finishing the assembling process.

As described above, the brush holder assembly according to a preferred embodiment of the comprises a brush holder 100, a pair of brushes 200 having a spring 220 respectively, a non-metallic plate 700, a silicone member 600 supplied by spraying silicon, and a cover 500, wherein the assembling process of the non-metallic plate 700 and the spraying method of the silicone are newly provided in the present invention.

Therefore, in the present invention, both the brush insertion holes can be commonly blocked by using a single non-metallic plate instead of using two metallic connection pieces separately, so that the assembling work and lead line soldering process are simplified.

In particular, in the present invention, the silicone is supplied and cured on the non-metallic plate, so that it is possible to achieve very tight sealing and to perfectly prevent the penetration of moisture and foreign substances.

As the present invention may be embodied in several forms without departing from the spirit or essential characteristics thereof, it should also be understood that the above-described examples are not limited by any of the details of the foregoing description, unless otherwise specified, but rather should be construed broadly within its scope as defined in the appended claims, and therefore all changes and modifications that fall within the meets and bounds of the claims, or equivalences of such meets and bounds are therefore intended to be embraced by the appended claims.

## Claims

1. A brush holder assembly of an alternator for a vehicle, comprising:
a brush holder (100) that includes a circular slip ring insertion part (140), a brush box (110) connected on the brush holder (100) and having a pair of brush insertion holes (150) and electrode pieces (130) inside, and a connection part (120) connected at one side of the brush box (110);
a pair of brushes (200) that are inserted into the brush box (110) of the brush holder (100) and provided with lead lines (210) at rear ends, wherein the lead lines (210) are provided with compression springs and are connected to the electrode pieces (130);
a non-metallic plate (700) having lead line holes (710) for passing the lead lines (210) is provided on the brush insertion holes (150), wherein the non-metallic plate (700) induces the lead lines (210) of the brushes (200) into the insulation state with respect to the electrode pieces (130) provided at a side of the brush box (110);
a cover (500) for covering an upper side of the non-metallic plate (700) to be fixed at the brush box (110)
**characterized in that** the non-metallic plate (700) includes grommets (720) provided to the lead line holes (710), and
a silicone member (600) adapted to sealingly cover an upper side of the non-metallic plate (700) for blocking the brush insertion holes (150) of the brush box (110).

2. The assembly of claim 1, wherein the grommets (720) are formed in the horn shape of which a lower side is wide, and an upper side is narrow, so that the lead lines (210) can be easily inserted and lifted.

## Patentansprüche

1. Bürstenhalteranordnung einer Lichtmaschine für ein Fahrzeug, die umfasst:
einen Bürstenhalter (10), der einen kreisförmigen Schleifringeinsetzabschnitt (140), einen mit dem Bürstenhalter (100) verbundenen Bürstenkasten (110) mit einem Paar Bürsteneinsetzlöcher (150) und Elektrodenteilen (130) innerhalb sowie einen Verbindungsabschnitt (120), der auf einer Seite des Bürstenkastens (110) verbunden ist, enthält;
ein Paar Bürsten (200), die in den Bürstenkasten (110) des Bürstenhalters (100) eingesetzt sind und an hinteren Enden mit Leitungen (210) versehen sind, wobei die Leitungen (210) mit Druckfedern versehen und mit den Elektrodenteilen (130) verbunden sind;
eine nicht metallische Platte (700) mit Leiterlöchern (710) für den Durchgang der Leiter (210), die an den Bürsteneinsetzlöchern (150) vorgesehen sind, wobei die nicht metallische Platte (700) die Leiter (210) der Bürsten (200) in Bezug auf die auf einer Seite des Bürstenkastens (110) vorgesehenen Elektrodenteile (130) isoliert;
eine Abdeckung (500), um eine Oberseite der nicht metallischen Platte (700) abzudecken, die am Bürstenkasten (110) zu befestigen ist,
**dadurch gekennzeichnet, dass** die nicht metallische Platte (700) Durchführungstüllen (720) aufweist, die an den Leiterlöchern (710) vorgesehen sind, und
ein Silikonelement (700) dazu ausgelegt ist, eine Oberseite der nicht metallischen Platte (700) dicht abzudecken, um die Bürsteneinsetzlöcher (150) des Bürstenkastens (110) zu versperren.

2. Anordnung nach Anspruch 1, wobei die Durchführungstüllen (720) hornförmig ausgebildet sind, wobei eine Unterseite weit ist und eine Oberseite eng ist, so dass die Leiter (210) einfach eingesetzt und angehoben werden können.

## Revendications

1. Ensemble porte-balais d'un alternateur pour un véhicule, comprenant :
un porte-balais (100) qui inclut une partie d'insertion à bague coulissante circulaire (140), un boîtier de balais (110) connecté au porte-balais (100) et ayant une paire de trous d'insertion de balais (150) et des pièces d'électrodes (130) à l'intérieur, et une partie de connexion (120) connectée sur un côté du boîtier de balais (110) ;
une paire de balais (200) qui sont insérés dans le boîtier de balais (110) du porte-balais (100) et équipés de lignes de liaison (210) aux extrémités postérieures, dans lequel les lignes de liaison (210) sont pourvues de ressorts de compression et sont connectées aux pièces d'électrodes (130) ;
une plaque non métallique (700) ayant des trous (710) pour lignes de liaison afin de faire passer les lignes de liaison (210) est prévue sur les trous d'insertion de balais (150), dans lequel la plaque non métallique (700) induit les lignes de liaison (210) des balais (200) dans l'état d'isolation par rapport aux pièces d'électrodes (130) prévues sur un côté du boîtier de balais (110) ;
une couverture (500) pour couvrir un côté supérieur de la plaque non métallique (700) à fixer sur le boîtier de balais (110),
**caractérisé en ce que** la plaque non métallique (700) inclut des joints (720) prévus vers les trous (710) pour lignes de liaison, et
un élément en silicone (600) adapté à couvrir de façon étanche un côté supérieur de la plaque non métallique (700) pour bloquer les trous d'insertion de balais (150) du boîtier de balais (110).

2. Ensemble selon la revendication 1, dans lequel les joints (720) sont formés sous la forme de cornes dont un côté inférieur est large et un côté supérieur est étroit, de sorte que les lignes de liaison (210) peuvent être aisément insérées et soulevées.
